# EUROPEAN PATENT APPLICATION

(11) **EP 2 666 413 A1**
(43) Date of publication of application: **27.11.2013**
(21) Application number: 12169102.6
(22) Date of filing: 23.05.2012
(51) Int. Cl.: A61B 6/00, G06T 11/00

(54) **Bone density measurement**

(71) Applicant: Stryker Trauma GmbH, 24232 Schönkirchen/Kiel (DE)
(72) Inventor: Simon, Bernd, 24107 Kiel (DE); Blau, Arno, 79112 Freiburg (DE)
(74) Representative: Maiwald Patentanwalts GmbH

(57) **Abstract**

A method and device for determining a tissue density is provided, the method comprising the steps of receiving an X-ray image of a region of interest and information about a theoretical distribution of more or less dense tissue in the region of interest, comparing a pixel of the X-ray image related to tissue with a pixel related to a reference body, estimating an actual distibution of tissue density based on the received information and the result of the pixel comparison.

## Description

### FIELD OF THE INVENTION

The invention relates to the field of computer assisted surgery (CAS). Particularly, the invention relates to a computer software based method for identifying a density distribution of a tissue like, for example, bone. Furthermore, the invention relates to a corresponding device.

### BACKGROUND OF THE INVENTION

In the field of radiology, images are generated by means of a system including an X-ray source and an X-ray detector, wherein radiation is emitted from the X-ray source and is detected by the X-ray detector after passing through an object. The X-ray detector may be an array of sensing elements for converting the radiation into a signal which can be processed by an appropriate processing unit. Based on these signals from the sensing elements, wherein each of these sensing elements may be related to a pixel, the processing unit generates an X-ray image which can be shown on a monitor or which can be printed. Accordingly, such an image is a two dimensional projection image and has the drawback that it does not include any depth information.

To achieve three dimensional information of an object, a plurality of images can be generated from different viewing angles. An appropriate processing unit may generate a 3D visualization on the basis of such images. However, this has the drawback that a patient must be exposed to a high amount of radiation to achieve a 3D visualization.

### SUMMARY OF THE INVENTION

It may be seen as an object to provide means for determining a density distribution of a tissue. It may be seen as a further object to reduce the amount of radiation to which a patient has to be exposed. These and other objects will be solved by the subject-matter of each of the independent claims. Further embodiments are described in the respective dependent claims.

According to a first aspect, a method for determining a tissue density is provided comprising, in general, the steps of receiving an X-ray image of a region of interest and information about a theoretical distribution of more or less dens tissue in the region of interest, comparing a pixel of the X-ray image related to tissue with a pixel related to a reference body, estimating an actual distibution of tissue density based on the received information and the result of the pixel comparison.

The X-ray projection image may be generated from a first imaging direction, wherein this image including a region of interest of a subject and the reference body. The reference body may include a predetermined density and thus a predetermined X-ray attenuation. The X-ray projection image may comprise a plurality of pixels, wherein each of the pixels is associated with one X-ray beam path and represents an attenuation of the X-ray beam along its path through the subject. Usually, a distinct grey level of one pixel in X-ray image corresponds to a distinct level of attenuation of the X-ray beam.

Further, the received information may include a theoretical distribution of harder, i.e. more dens tissue and softer, i.e. less dens tissue which can be expected along the path of the X-ray beam.

It will be understood that the signal of one sensing element of the X-ray detector corresponds to an X-ray attenuation of the X-ray beam through an object. Furthermore, one sensing element may correspond to one pixel of an X-ray image. Accordingly, the remaining X-ray radiation (emitted X-ray radiation minus attenuation of the same) as sensed by one sensing element can be assigned to a specific grey level for a pixel corresponding to the sensing element.

According to an embodiment, the grey level of one of the pixels related to the region of interest is compared with the grey level of one of the pixels related to the reference body, so that an actual distribution of harder tissue and of softer tissue along the path of the X-ray beam related to the pixel related to the region of interest can be estimated, based on the compared grey levels representing X-ray beam attenuations, and the theoretical distribution of harder and softer tissue.

A distruibution of the density of differing tissues is thus determined along the path of an X-ray beam associated with one pixel.

According to an embodiment, the X-ray image comprises a plurality of pixels in the region of interest and the steps of comparing and of estimating are performed for each pixel of the region of interest.

Performing the method on a plurality of pixels will result in a three dimensional density distribution. It is thus possible to estimate a density distribution of a three dimensional object on the basis of only one two dimensional projection image.

According to another embodiment, the method further comprises the step of receiving a second X-ray projection image generated from a second imaging direction which is different to the first imaging direction, wherein the second image may also include the region of interest. Taking into account the second X-ray image, the estimation of the actual distribution of harder tissue and softer tissue may be performed for each voxel of the region of interest. As used herein, the term "voxel" encompasses a point in a three dimensional space, whereas the term "pixel" denotes a point in a two dimensional space.

Based on the second X-ray image, it may be possible to verify and, if necessary, revise the three dimensional density distribution as estimated based on the first X-ray image.

Thus, a three dimensional density distribution may be estimated based on a plurality, i.e. at least two X-ray images generated from different imaging directions, and based on information concerning a theoretical distribution of harder and softer tissue. That is, not only the typical distribution but also actual values can be taken into account for the estimation of the density distribution in a three dimensional space. For example, a pathologically changed density of only a portion in the region of interest can be identified by an estimation based on at least two X-ray images from different directions.

According to a further embodiment, the method further comprises the step of identifying bone tissue in the region of interest, wherein an actual distribution of harder bone tissue and softer bone tissue is estimated.

Based on at least one projection image, a density distribution may be estimated taking into account a typical distribution of more or less dens tissue in a region of interest like a bone, i.e. for example a tissue distribution present in a bone model. As use herein, the term "bone model" encompasses, for example, a 3D model of a bone. The bone model may be generated based on at least one 3D scan of at least one real bone of the same kind, for example a femur or humerus, and/or by forming an average from a plurality of 3D scans. An exemplary utilization of bone models is described in 'Evidence based development of a novel lateral fibula plate (VariAX Fibula) using a real CT bone data based optimization process during device development' of A.P. Schulz et al. (The Open Orthopaedics Journal, 2012, 6, 1-7), the content of which is incorporated herein by reference.

A database may contain several models of each bone (e.g. tibia, femur, humerus) including bone models of different ages, genders and individual sizes. Various aspects like for example a distribution of differing tissues may be taken from a 3D model. Furthermore, an attenuation value along a projection path may be calculated based on a bone model, providing a value of an attenuation which can be expected at a distinct position when a bone would be imaged from a specific direction.

According to another embodiment, the method further comprises the step of visualizing the density distribution on a monitor. For example, the different density values may be visualized by different colors, by different grey levels, by more or less narrow array of points or by more or less narrow lines of hatchings. Furthermore, portions having a density which differs from a theoretically expectable density may be high-lighted by for example flashing.

According to an embodiment, the reference body includes a plurality of portions with different predetermined densities. By way of this, a plurality of values, i.e. steps of grey levels corresponding to density values can be compared with the values of the at least one pixel of the imaged region of interest.

According to a second aspect, a device for determining a tissue density is provided comprising a processing unit adapted to perform the steps of the above described method.

According to an embodiment, the device comprises a storage device providing a database of for example bone models. It will be understood, that such storage means may also be provided in a network to which the device may be connected and information related to the bone model, i.e. different types of models and density values thereof, may be received over that network.

According to another embodiment, the device further comprises an X-ray imaging unit for generating an X-ray image, wherein the X-ray imaging unit may be capable of generating X-ray images from different imaging directions.

According to an embodiment, the device further comprises an input device for manually identifying a region of interest. Such input means may be for example a computer keyboard, a computer mouse or a touch screen, to control a pointing device like a cursor on a monitor screen which may also be included in the device.

According to a third aspect, a computer program is provided including sets of instructions which when executed on a processing unit of an above described device, cause the device to perform the steps of the above described method.

A corresponding computer program may preferably be loaded into a work memory of a data processor. The data processor or processing unit may thus be equipped to carry out at least a part of the method. Further, the invention relates to a computer-readable medium such as a CD-ROM at which the computer program may be stored. However, the computer program may also be presented over a network like the World Wide Web and can be downloaded into the working memory of the data processor from such a network.

It has to be noted that embodiments are described with reference to different subject-matters. In particular, some embodiments are described with reference to method type claims (computer program) whereas other embodiments are described with reference to apparatus type claims (device). However, a person skilled in the art will gather from the above and the following description that unless other notified in addition to any combination of features belonging to one type of subject-matter also any combination between features relating to different subject-matters is considered to be disclosed with this application.

The aspects defined above and further aspects, features and advantages of the present invention can also be derived from the examples of the embodiments to be described hereinafter and are explained with reference to examples of embodiments also shown in the figures, but to which the invention is not limited.

### BRIEF DESCRIPTION OF THE DRAWINGS

Fig. 1 shows a flow chart of steps of an embodiment of the method.
Fig. 2 shows a schematical illustration of a system according to one embodiment.
Fig. 3A and 3B illustrate exemplary embodiments of reference bodies.
Fig. 4 illustrates an exemplary positioning of a reference body so that the reference body may be imaged together with a region of interest.
Fig. 5A is a detail view of the schematically image of Fig. 5B showing a reference body together with a region of interest.
Fig. 6 shows several X-ray beams passing through a region of interest from different directions.
Fig. 7 is a schematically visualization of a measured and an expected attenuation of pixels of an image generated in x and y direction, as indicated in Fig. 6.

Throughout the drawings, the same reference numerals and characters, unless otherwise stated, are used to denote like features, elements, components, or portions of the illustrated embodiments. Moreover, while the present disclosure will now be described in detail with reference to the figures, it is done so in connection with the illustrative embodiments and is not limited by the particular embodiments illustrated in the figures.

### DETAILED DESCRIPTION OF THE EMBODIMENTS

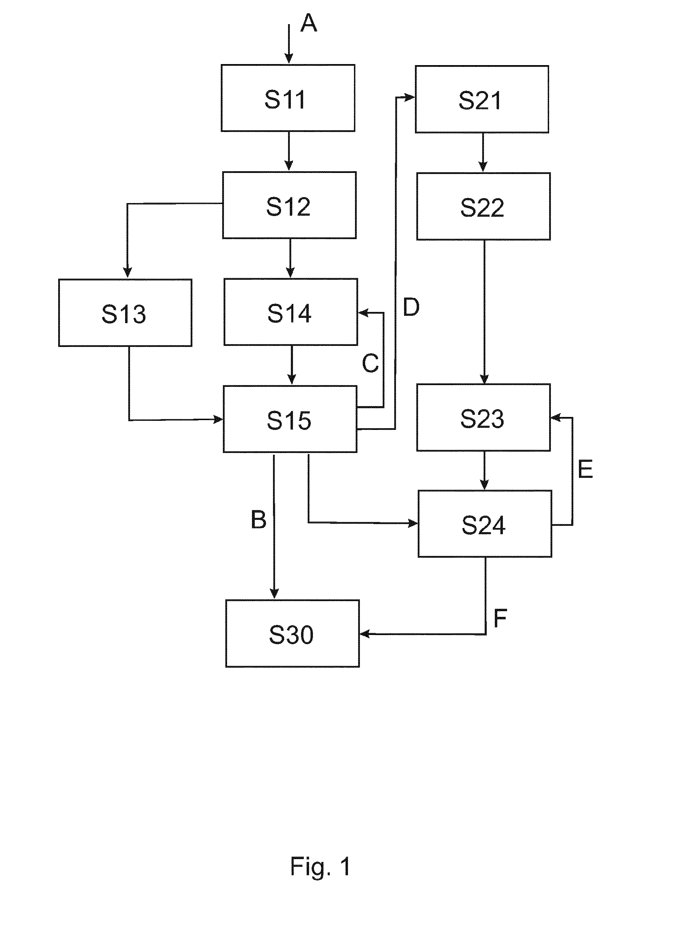

The flow-chart in Fig. 1 illustrates the principle of the steps performed in accordance with one embodiment. It will be understood that the steps described, are major steps, wherein these major steps might be differentiated or divided into several sub-steps. Furthermore, there might be also sub-steps between these major steps.

As depicted in Fig. 1, several possibilities of performing a method are provided.

Starting at arrow A, an image is received for example from an X-ray imaging unit in step S11, the image being generated from a first direction and shows a region of interest as well as a reference body. In step S12, the region of interest, for example a portion of a bone is identified in the received image.

In step S 13, information regarding the distribution of density in the identified bone portion is received. For example, a bone model of a long bone may be received including an average distribution of more or less dens tissue, i.e. of harder bone tissue present in a corticalis of the bone and softer bone tissue present in a marrow channel of the bone. Knowing the tissue distribution leads to an attenuation value which can be expected.

In step S 14, the value of a pixel of the region of interest is compared with a value of a pixel of the reference body, wherein each of such pixel values represents an overall attenuation of the X-ray beam impinging a respective sensing element of an X-ray detector.

In step S 15, a distribution of density along the path of an X-ray beam is estimated based on the comparison of the pixel value (S 14) and the bone model (S 13).

Following arrow B, in a case in which an estimation with respect to only one pixel is considered as sufficient, the X-ray image together with the density information is visualized in step S30.

Following arrow C, in a case in which a plurality of pixels should be taken into account, steps S 14 and S15 are performed again for each of that pixels. By repeating this part of the method, an estimation of a three dimensional distribution of the density can be achieved on the basis of the X-ray image generated in step S11.

Following arrow D, step S21 is performed next, i.e. a further image is received for example from the X-ray imaging unit, the image being generated from a second direction showing also the region of interest. In step S22, the region of interest is identified in the received image.

In step S23, the value of a pixel of the region of interest is compared with a value of a pixel of the reference body. In step S24, a three dimensional distribution of density is estimated based on the comparison of the pixel value of the second image (S23) and the distribution as estimated in step S 15 taking into account the bone model (S13).

Following arrow E, steps S23 and S24 are performed at least twice, for a plurality of pixels of the second X-ray image.

Following arrow F, one of the X-ray images together with the verified or revised, thus improved density information is visualized in step S30.

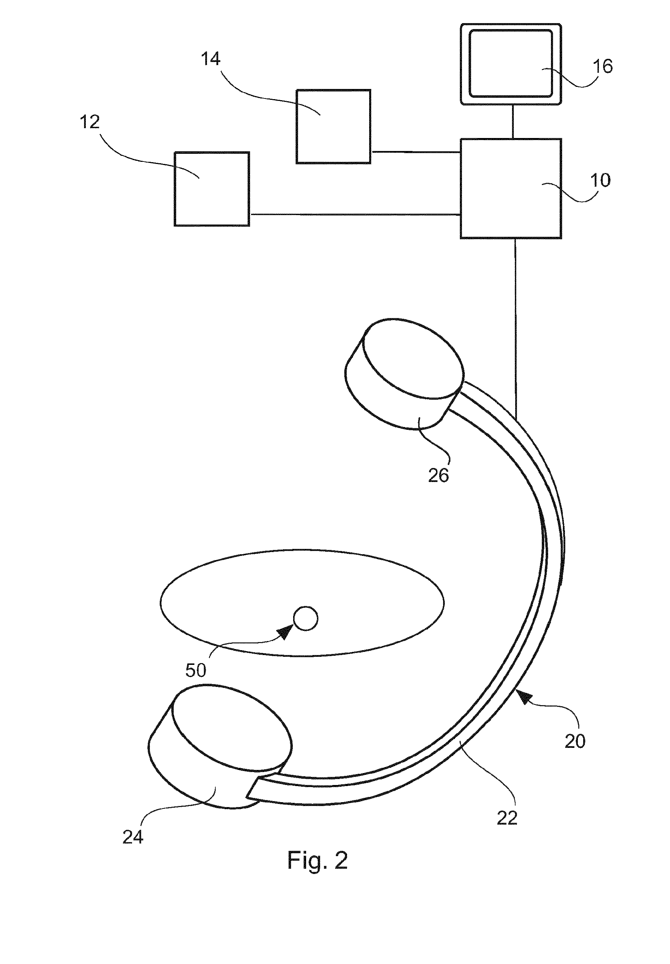

Fig. 2 shows an exemplary embodiment of a device. Substantially necessary for performing the steps according to the method, the device comprises a processing unit 10 and a monitor 16.

The exemplary imaging device 20 includes an X-ray source 24, and an X-ray detector 26, wherein these two devices are mounted on a C-arm 22. It will be understood that the device may also comprise another non-invasive imaging modality like a computer tomography device as imaging device instead of or additional to the shown C-arm based X-ray device, to generated individual projection images.

Furthermore, the device in Fig. 2 includes an input device 14, by means of which for example a manual determination of a position on a bone surface may be performed. Also shown is a connection to a database 12, located for example in a network.

Finally, there is shown a region of interest 50. Within said region, for example a bone of a patient may be located, a density distribution of which may be of interest.

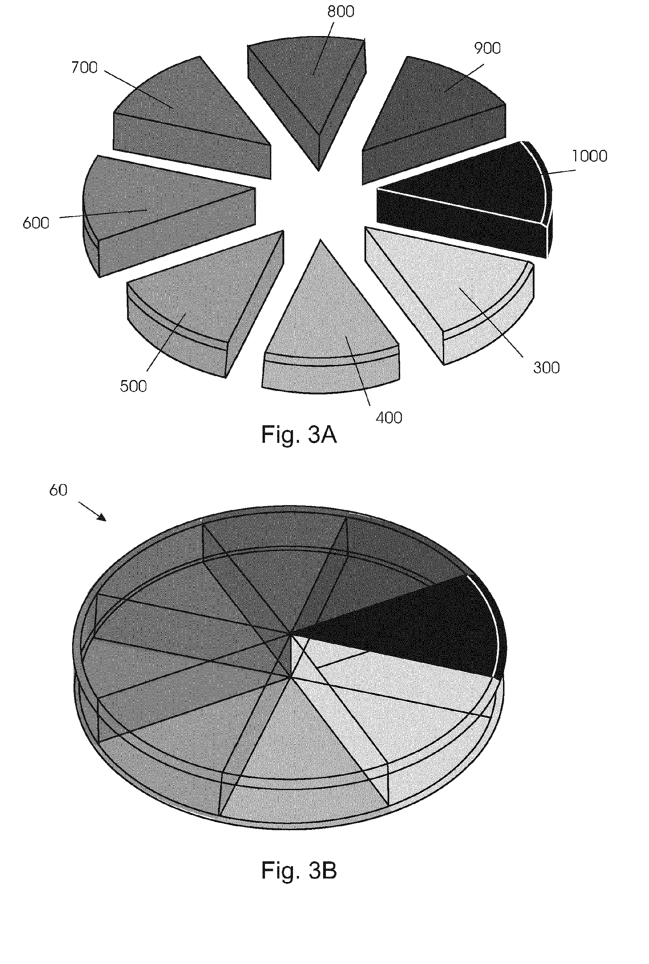

Fig. 3A and Fig. 3B show exemplary embodiments of reference bodies. In Fig. 3A, eight reference bodies are depicted each formed like a piece of cake. It will be understood that one reference body may have any other form, for example rectangular, circular, or cubical. Preferably, a reference body does not have any sharp edges or tips to avoid any injury of a person.

As illustrated by different grey levels in Fig. 3A, each of the reference bodies may comprise a material with a distinct X-ray attenuation. For example, reference body 300 may be made from a less dens material as each of the other reference bodies 400, 500, 600, 700, 800, 900 and 1000. To illustrate this, reference body 300 is shown in Fig. 3A with the brightest grey level. Consequently, reference body 1000 may be made from a more dens material as each of the other reference bodies, thus being shown with the darkest grey level. It will be understood that the density of a material affects the X-ray attenuation, i.e. the possibility for an X-ray beam to pass the material, for example without being scattered.

Fig. 3B shows an embodiment of a reference body 60 having a plurality of segments with different X-ray attenuation. This reference body comprises the form of a disk which may be made by a plurality of separate pieces each with a material having a different X-ray attenuation. It will be understood that also a reference body 60 with a plurality of different X-ray attenuations may be arbitrarily formed, for example like a cube or a cylinder.

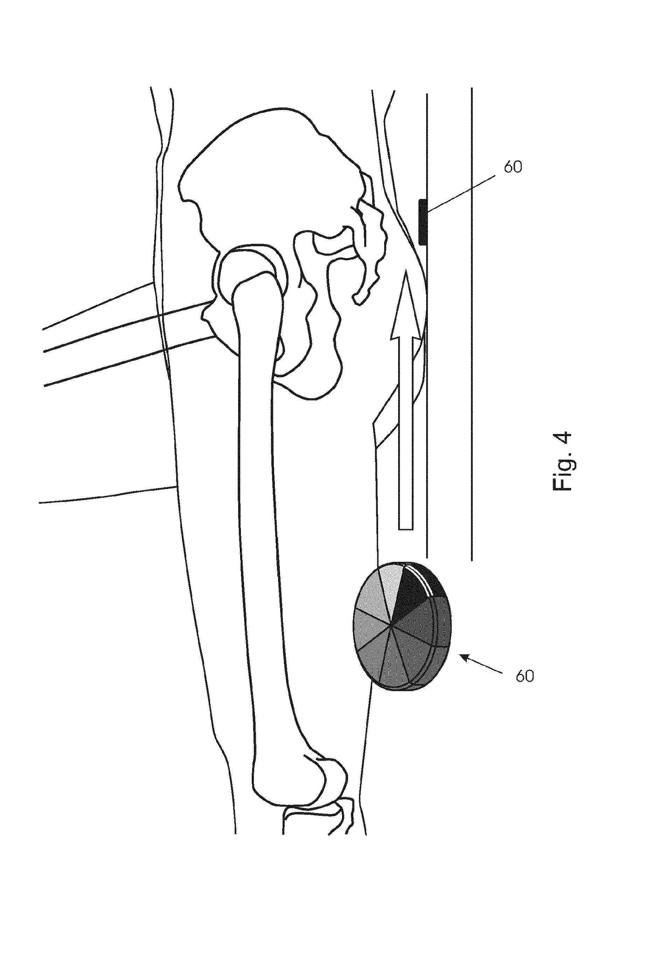

In Fig. 4, a possible positioning of a reference body 60 is shown. As indicated by the arrow, the reference body 60 may be laid under a body of a patient on a table of an X-ray system. The position should be in the vicinity of the region of interest, i.e. the portion of a body which is intended to be imaged by means of the X-ray system. Furthermore, the position should be within the field of view of the X-ray system to ensure that the reference body will be imaged together with the region of interest.

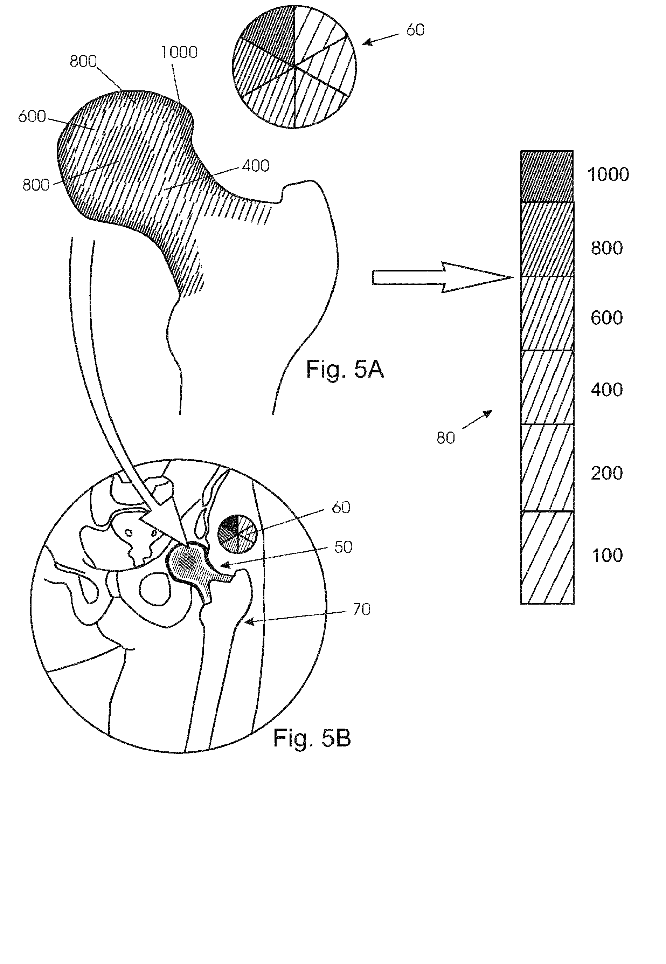

In the example shown in Fig. 4, it may be intended to image the hip joint from an anterior to posterior direction, i.e. from above (when the patient lie on the back). An exemplary image of a hip joint also including a reference body is shown in Fig. 5B.

It is noted that the reference body does not have to be in contact with the patient during the imaging.

Fig. 5B is a schematically illustration of an X-ray image including a femur 70, wherein the head 50 of the femur 70 may be considered as region of interest, as well as a reference body 60.

The head 50 of the femur 70 is also shown in figure 5A which is an enlarged detail view of the region of interest and the reference body of figure 5B. The head of the femur includes hatchings, wherein these hatchings represent areas with different densities. For example, the area 1000 forming substantially the corticalis of the bone includes a high density as indicated in the scale 80 at the right side of figure 5A.

According to the illustrated example, the density firstly decreases from the edge area 1000, i.e. the corticalis, in the direction to the area in the middle of the head. That is, adjacent the edge area 1000, an area 800 and then an area 600 having less density are present. However, in the middle of the head of the femur a further area 800 with an relatively increased density can be seen, whereas in the middle of the neck of the femur an area 400 with further decreased density is present.

It is noted that the reference signs 100, 200, 300, 400, 500, 600, 700, 800, 900, and 1000, as used in figures 3A and 5A, both may denote an area and may indicate an order of magnitude of the density.

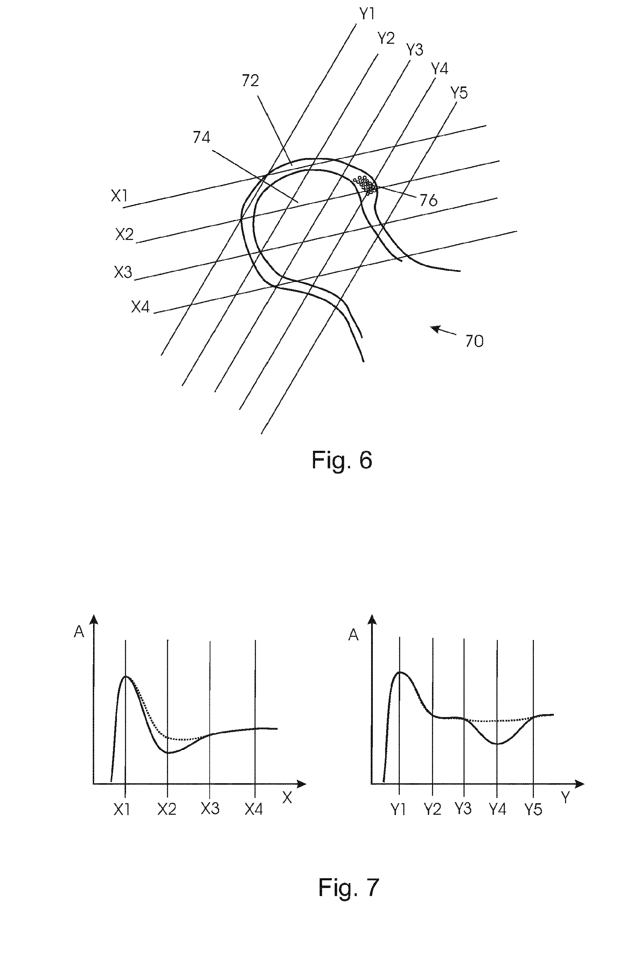

In Fig. 6, a femur head 70 is shown but simplified in that it only includes a corticalis 72 and marrow 74. In this example, the corticalis includes a portion 76 which may be pathologically changed. Further shown in figure 6 are several x-ray beams a first group of which (X1, X2, X3, X4) passes through the head of the femur 70 from a first direction and a second group of which (Y1, Y2, Y3, Y4, Y5) passes through the head of the femur 70 from a second direction.

As can be seen from Fig. 7, the X-ray attenuation (A) of beam X1 is higher than that of for example beam X3, although beam X1 only passes through corticalis tissue and beam X3 passes through both corticalis and marrow. As the attenuation (A) in the harder corticalis is higher than in the softer marrow, an X-ray beam has to pass through more marrow than corticalis to be subjected to a comparable attenuation. Assuming that the femur head in Fig. 6 has been imaged only by means of the beams X1 to X4, in a first step, a performing of one method would result in an indication of the pixel associated with beam X2 as deviating from an expected density value, wherein the expected values are indicated by the dotted lines in Fig. 7 and the measured values are indicated as continuous lines in Fig. 7. With such a result, it can be diagnosed that a portion with mutated tissue exists within the femur head, but not as to whether this portion is in the corticalis on the left side, the marrow along the beam path or in the corticalis on the right side of Fig. 6.

With a further imaging in a direction of the beams Y1 to Y5, the first result can be improved, since a density value corresponding to beam Y4 deviates from an expectable attenuation (A) value and thus density value, as shown in Fig. 7. With this additional information, it can be assessed where the portion including mutated tissue is located.

In accordance with one method, it is possible to visualize a view like that in Fig. 6, with a portion 76 being high-lighted, although only projection images perpendicular to the viewing direction has been generated.

While embodiments has been illustrated and described in detail in the drawings and afore-going description, such illustrations and descriptions are to be considered illustrative or exemplary and not restrictive, the invention is not limited to the disclosed embodiments.

Other variations to the disclosed embodiments can be understood and effected by those skilled in the art in practising the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims.

The mere fact that certain measures are recited and mutually different dependent claims does not indicate that a combination of these measures cannot be used to advantage. The computer program may be stored/distributed on a suitable medium such as an optical storage medium or a solid-state medium supplied together with or as a part of another hardware, but may also be distributed in other forms, such as via the Internet or other wired or wireless telecommunication systems. Any reference signs in the claims should not be construed as limiting the scope.

### LIST OF REFERENCE SIGNS

- 10: processing means
- 12: database
- 14: input device
- 16: monitor
- 20: imaging device
- 22: C-arm
- 24: X-ray source
- 26: X-ray detector
- 50: region of interest
- 60: reference body
- 70: femur
- 72: corticalis of femur head
- 74: bone tissue within femur head
- 76: portion of mutated tissue
- 80: density scale
- 100, 200, 300, 400, 500, 600, 700, 800, 900, 1000: area and/or density value

## Claims

1. A method for determining a tissue density, the method comprising the steps of:
receiving a first X-ray projection image generated from a first imaging direction, the first image including a region of interest (50) of a subject and a reference body (60), the reference body having a predetermined density,
wherein the X-ray projection image comprises a plurality of pixels, wherein each of the pixels is associated with one X-ray beam and represents an attenuation of the X-ray beam along its path through the subject,
receiving information about a theoretical distribution of harder tissue and softer tissue to be expected along the path of the X-ray beam,
comparing the X-ray attenuation of one of the pixels related to the region of interest with one of the pixels related to the reference body, and
estimating an actual distribution of harder tissue and of softer tissue along the path of the X-ray beam related to the pixel related to the region of interest, based on the compared X-ray beam attenuation and the theoretical distribution of harder and softer tissue.

2. The method of claim 1, wherein the steps of comparing and of estimating are performed for each pixel of the region of interest.

3. The method of claim 2, further comprising the step of
receiving a second X-ray projection image generated from a second imaging direction which is different to the first imaging direction, the second image including the region of interest,
wherein the estimation of the actual distribution of harder tissue and softer tissue is based on the compared X-ray beam attenuation and the theoretical distribution of harder and softer tissue and is performed for each voxel of the region of interest.

4. The method of claim 1, further comprising the step of identifying bone tissue in the region of interest, wherein an actual distribution of harder bone tissue and softer bone tissue is estimated.

5. The method of claim 1, further comprising the step of visualizing the distribution on a monitor.

6. The method of claim 1, wherein the reference body includes a plurality of portions with different predetermined densities.

7. A device for determining a tissue density, the device comprising:
a processing unit (10) adapted to perform the steps of the method of claim 1.

8. The device of claim 7, further comprising an x-ray imaging unit (20) for generating an X-ray image.

9. The device of claim 8, wherein the X-ray imaging unit is capable of generating X-ray images from different imaging directions.

10. The device of claim 7, further comprising a reference body (60) having at least one portion with a predetermined density.

11. The device of claim 7, further comprising means for manually identify a region of interest (50).

12. The device of claim 7, further comprising a monitor (16) for a visualization of the estimated distribution of harder and softer tissue in a region of interest.

13. A computer program including sets of instructions which when executed on a processing unit (10) of a device according to claim 7, cause the device to perform the steps of the method according to claim 1.
